(19) Europäisches Patentamt / European Patent Office / Office européen des brevets

(11) EP 1 844 792 A1

(12) **EUROPEAN PATENT APPLICATION**
published in accordance with Art. 158(3) EPC

(43) Date of publication:
**17.10.2007 Bulletin 2007/42**

(21) Application number: **05816775.0**

(22) Date of filing: **13.12.2005**

(51) Int Cl.:
***A61K 45/00*** *(2006.01)* ***A61K 31/485*** *(2006.01)*
***A61P 1/10*** *(2006.01)*

(86) International application number:
**PCT/JP2005/022822**

(87) International publication number:
**WO 2006/064780 (22.06.2006 Gazette 2006/25)**

(84) Designated Contracting States:
**AT BE BG CH CY CZ DE DK EE ES FI FR GB GR HU IE IS IT LI LT LU LV MC NL PL PT RO SE SI SK TR**

(30) Priority: **14.12.2004 JP 2004360966**
**04.02.2005 JP 2005028927**
**08.04.2005 JP 2005111912**
**11.10.2005 JP 2005296045**

(71) Applicant: **Shionogi Co., Ltd.**
**Osaka-shi, Osaka 541-0045 (JP)**

(72) Inventors:
- **SUZUKI, Tsutomu**
  **Yokohama-shi, Kanagawa 2350019 (JP)**
- **SAWADA, Takuko,**
  **c/o Shionogi & Co., Ltd.**
  **Osaka-shi, Osaka 5530002 (JP)**
- **ISHIHARA, Yasunobu,**
  **c/o Shionogi & Co., Ltd.**
  **Osaka-shi, Osaka 5530002 (JP)**

(74) Representative: **Kador & Partner**
**Corneliusstrasse 15**
**80469 München (DE)**

(54) **THERAPEUTIC AGENT FOR CONSTIPATION**

(57) A therapeutic and/or prophylactic agent for constipation induced by a compound having an opioid μ receptor agonist activity, which agent comprises as an effective ingredient a compound having an opioid δ receptor antagonist activity, e.g., a compound of Formula (I):

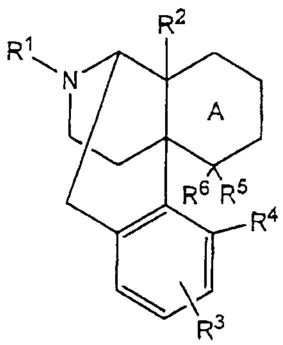

(I)

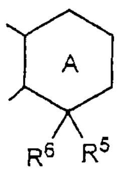

(II)

EP 1 844 792 A1

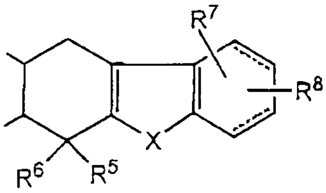 ,

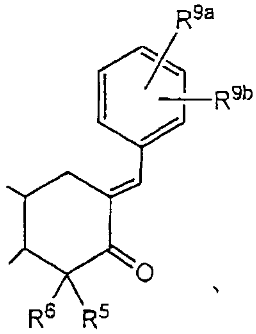 , (III)

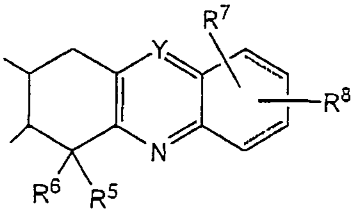 or 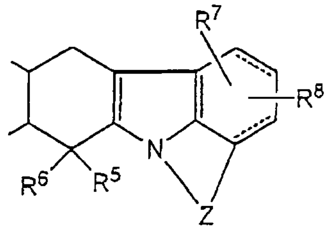

(wherein $R^1$ represents hydrogen, lower alkyl, cycloalkyl lower alkyl or the like; $R^2$ and $R^3$ independently represent hydrogen, hydroxy or the like; $R^4$ is hydrogen, hydroxy or the like; $R^5$ is hydrogen; $R^4$ and $R^5$ may optionally form -O- or the like; $R^6$ represents hydrogen, lower alkyl or the like (wherein X represents -O- or -N($R^{10}$)-or the like; $R^7$, $R^8$, $R^{9a}$ and $R^{9b}$ independently represent hydrogen, lower alkyl, lower alkoxycarbonyl or the like; r represents an integer of 0 to 5; Y represents -CH- or the like; Z represents a crosslinkage composed of 2 to 5 atoms)
or a pharmaceutically acceptable salt thereof or a solvate of either.

## Description

### Technical Field

The present invention relates to a therapeutic and/or prophylactic agent for constipation in which opioid μ receptor is involved, especially, which is induced by a compound having a μ agonist activity.

### Background Art

Opioid μ receptor agonists such as morphine are used as very effective analgesics for patients suffering from cancer pain. However, they induce strong vomiting, nausea, constipation, urinary retention, itching and the like as side effects. Although various antiemetics and anti-constipation drugs are clinically used, none of them exhibits sufficient effects, so that an excellent agent for reducing the side effects is demanded for the improvement of QOL of the patients. As the pharmaceuticals whose indication is dysfunction of digestive tract or constipation caused by administration of a nacrotic analgesic, methylnaltrexone bromide (MNTX), alvimopan and the like, which are opioid μ receptor antagonists, are now being developed.

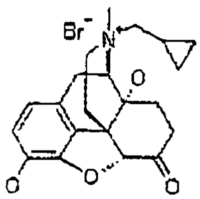

methylnaltrexone bromide

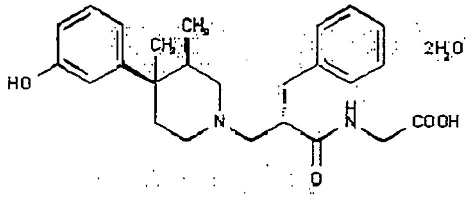

alvimopan

Patent Literature 1 discloses that naloxone, naltrexone and the like are effective for amelioration of dysfunction of gastrointestinal activc function .

Patent Literatures 2 to 4 and Non-patent Literature 1 disclose that MNTX and derivatives thereof, naloxone, N-methylnaloxone and the like are effective for amelioration of side effects induced by an opioid, and constipation is listed as an example of the side effects.

Patent Literatures 5 to 7 disclose that piperidine-N-alkylcarboxylate derivatives which are opioid μ antagonists are effective for irritable bowel syndrome, constipation, ileus and the like.

Although the compound (I) of the present invention and analogues thereof are disclosed in Patent Literatures 8 to 20 and Non-patent Literatures 2 and 3, none of them discloses the therapeutic or prophylactic effect thereof for constipation.

Patent Literature 1: International Patent Publication WO83/03197
Patent Literature 2: International Patent Publication WO99/22737
Patent Literature 3: International Patent Publication WO01/032180
Patent Literature 4: International Patent Publication WO98/25613
Patent Literature 5: Japanese Laid-open Patent Application (Kokai) No. H5-97806
Patent Literature 6: International Patent Publication WO01/037785
Patent Literature 7: International Patent Publication WO01/42207
Patent Literature 8: International Patent Publication WO89/00995
Patent Literature 9: International Patent Publication WO95/31463
Patent Literature 10: International Patent Publication WO94/07896
Patent Literature 11: International Patent Publication WO97/11948
Patent Literature 12: International Patent Publication WO02/42309
Patent Literature 13: International Patent Publication WO2004/007503
Patent Literature 14: International Patent Publication WO98/31684
Patent Literature 15: International Patent Publication WO94/14445
Patent Literature 16: International Patent Publication WO91/07966
Patent Literature 17: U.S. Patent No. 6,271,239
Patent Literature 18: International Patent Publication WO95/13071
Patent Literature 19: International Patent Publication WO93/21188

Patent Literature 20: U.S. Patent No. 6,476,044
Non-patent Literature 1: Journal of Pharmacology and Experimental Therapeutics, 300 (1), 118-123, (2002)
Non-patent Literature 2: Heterocycles 45, 2109-2112 (1997)
Non-patent Literature 3: Journal of Medicinal Chemistry 41, 4177-4180 (1998) Problems Which the Invention Tries to Solve

An object of the present invention is to provide a therapeutic and/or prophylactic agent for constipation induced by a compound having an opioid μ receptor agonist activity.

Means for Solving the Problems

The present invention provides:

(1) A therapeutic and/or prophylactic agent for constipation in which opioid μ receptor is involved, the agent comprising a compound having an opioid δ receptor antagonist activity;
(2) A therapeutic and/or prophylactic agent for constipation induced by a compound having an opioid μ receptor agonist activity, the agent comprising a compound having an opioid δ receptor antagonist activity;
(3) The therapeutic and/or prophylactic agent for constipation according to (1) or (2) above, wherein the compound having the opioid δ receptor antagonist activity has a higher affinity to opioid δ receptor than to opioid μ receptor;
(4) The therapeutic and/or prophylactic agent for constipation according to (1) or (2) above, wherein the compound having the opioid δ receptor antagonist activity is represented by Formula (I):

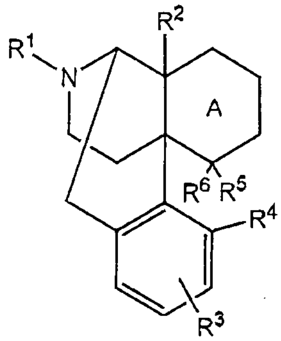

(I)

(wherein $R^1$ represents hydrogen, lower alkyl, cycloalkyl lower alkyl, cycloalkenyl lower alkyl, lower alkenyl, aryl, aryl lower alkyl, furyl lower alkyl or thienyl lower alkyl;
$R^2$ and $R^3$ independently represent hydrogen, hydroxy, lower alkoxy, lower alkenyloxy, aryl lower alkoxy, aryl lower alkenyloxy, acyloxy or lower alkoxy lower alkoxy;
$R^4$ represents hydrogen, hydroxy, lower alkoxy or acyloxy;
$R^5$ represents hydrogen;
$R^4$ and $R^5$ may optionally together form -O-, -S- or -CHz-;
$R^6$ represents hydrogen, lower alkyl, lower alkenyl, hydroxy lower alkyl, lower alkoxy lower alkyl, lower alkoxycarbonyl lower alkyl, aryl lower alkyl, aryl lower alkenyl, carboxy or lower alkoxycarbonyl;

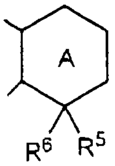 is 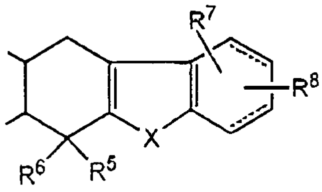 , 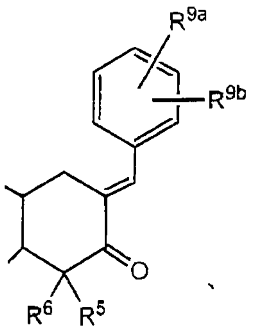 ,

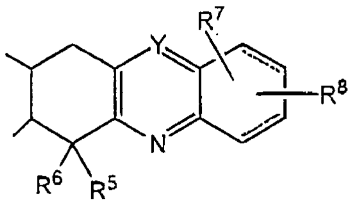 or 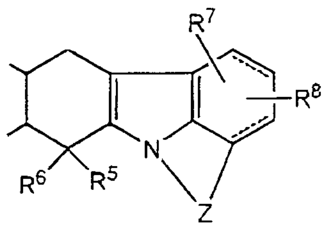

(wherein X represents -O-, -S-, -CH=CH- or -N($R^{10}$)-;

$R^7$, $R^8$, $R^{9a}$ and $R^{9b}$ independently represent hydrogen, halogen, nitro, lower alkyl, hydroxy, lower alkoxy, halogeno lower alkyl, hydroxy lower alkyl, halogeno lower alkoxy, hydroxy lower alkoxy, cyano, phenyl, isothiocyanato, $SR^{11}$, $SOR^{11}$, $SO_2R^{11}$, $(CH_2)_rOR^{11}$, $(CH_2)_rCOOR^{11}$, $SO_2NR^{12}R^{13}$, $CONR^{12}R^{13}$, $(CH_2)_rNR^{12}R^{13}$ or $(CH_2)_rN(R^{12})COR^{13}$;

$R^7$ and $R^8$ may optionally bind to adjacent carbon atoms in the ring to form a ring together with the carbon atoms, which ring may have a substituent(s); broken lines represent presence or absence of a bond, and in cases where the broken lines represent absence of the bond, $R^7$ and $R^8$ may optionally together form =O;

r represents an integer of 0 to 5;

$R^{10}$ represents hydrogen, lower alkyl, lower alkenyl, aryl lower alkyl, aryl lower alkenyl, acyl, lower alkylsulfonyl, arylsulfonyl, aryl lower alkylsulfonyl or acyl;

Y represents -N- or -CH-;

Z represents a crosslinkage composed of 2 to 5 atoms;

$R^{11}$ represents hydrogen or lower alkyl;

$R^{12}$ and $R^{13}$ independently represent hydrogen, lower alkyl or cycloalkyl lower alkyl)

or a pharmaceutically acceptable salt thereof or a solvate of either (hereinafter referred to as "compound (I)");

(5) The therapeutic and/or prophylactic agent according to )(4) above, wherein $R^1$ is cycloakyl lower alkyl;

$R^2$ and $R^3$ are hydroxy;

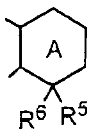 is 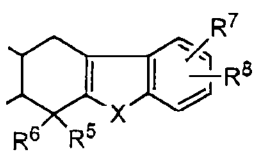 or 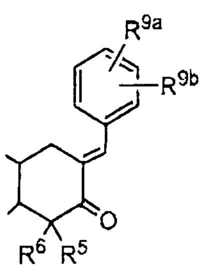

$R^4$ and $R^5$ together form -O-;

$R^6$ is hydrogen;

$R^7$, $R^8$, $R^{9a}$ and $R^{9b}$ independently are hydrogen, lower alkyl, carboxy or lower alkoxycarbonyl;

$R^{10}$ is hydrogen or lower alkyl; and

(6) The therapeutic and/or prophylactic agent according to any one of (2) to (5) above, wherein the compound having the opioid μ receptor agonist activity is morphine, oxycodone or a pharmaceutically acceptable salt thereof.
The present invention also provides:
(7) Use of a compound having an opioid δ receptor antagonist activity for the therapy and/or prophylaxis of constipation in which opioid μ receptor is involved;
(8) Usc of a compound having an opioid δ receptor antagonist activity for the therapy and/or prophylaxis of constipation induced by a compound having an opioid μ receptor agonist activity;
(9) Use of the compound represented by Formula (I) recited in (4) above or a pharmaceutically acceptable salt thereof or a solvate of either for the therapy and/or prophylaxis of constipation in which opioid μ receptor is involved;
(10) Use of a compound represented by Formula (I) recited in (4) above or a pharmaceutically acceptable salt thereof or a solvate of either for the therapy and/or prophylaxis of constipation induced by a compound having an opioid μ receptor agonist activity;
(11) A therapeutic and/or prophylactic method for constipation in which opioid μ receptor is involved, the method comprising administering a compound having an opioid δ receptor antagonist activity;
(12) A therapeutic and/or prophylactic method for constipation induced by a compound having an opioid μ receptor agonist activity, the method comprising administering a compound having an opioid δ receptor antagonist activity;
(13) A therapeutic and/or prophylactic method for constipation in which opioid μ receptor is involved, the method comprising administering the compound represented by Formula (I) recited in (4) above or a pharmaceutically acceptable salt thereof or a solvate of either;
(14) A therapeutic and/or prophylactic method for constipation induced by a compound having an opioid μ receptor agonist activity, the method comprising administering the compound represented by Formula (I) recited in (4) above or a pharmaceutically acceptable salt thereof or a solvate of either; and
(15) An analgesic comprising a compound having an opioid μ receptor agonist activity in combination with the compound represented by Formula (I) recited in (4) above or a pharmaceutically acceptable salt thereof or a solvate of either in an amount effective for the therapy and/or prophylaxis of constipation induced by the compound having the opioid μ receptor agonist activity.

Effects of the Invention

The compounds having an opioid δ receptor antagonist activity (hereinafter referred to as "the compound of the present invention") have a therapeutic and/or prophylactic activity against constipation in which opioid μ receptor is involved, especially against the constipation induced by a compound having an agonist activity, and arc useful as an agent for reducing side effects in the patients who are to receive or who are receiving a compound having an opioid μ receptor agonist activity.

Best Mode for Carrying out the Invention

In the present description, the term "halogen" includes fluorine, chlorine, bromine and iodine.
The above-described explanation is equally applied to the halogen moiety in "halogeno lower alkyl" and "halogeno lower alkoxy".
The term "lower alkyl" includes linear and branched alkyl groups having 1 to 10, preferably 1 to 6, more preferably 1 to 3 carbon atoms. Examples thereof include methyl, ethyl, *n*-propyl, isopropyl, *n*-butyl, isobutyl, *sec*-butyl, *tert*-butyl, *n*-pentyl, isopentyl, neopentyl, hexyl, isohexyl, *n*-heptyl, isoheptyl, *n*-ociyl, isooctyl, *n*-nonyl, *n*-decyl and the like.
The above-described explanation about the "lower alkyl" is equally applied to the lower alkyl moiety in "halogeno lower alkyl", "hydroxy lower alkyl", "cycloalkyl lower alkyl", "cycloalkenyl lower alkyl", "lower alkoxycarbonyl lower alkyl", "aryl lower alkyl", "furyl lower alkyl", "thienyl lower alkyl", "aryl lower alkylsulfonyl", "lower alkoxy lower alkyl", "lower alkylsulfonyl", "lower alkoxy", "lower alkoxy lower alkoxy", "halogeno lower alkoxy", "hydroxy lower alkoxy", "aryl lower alkoxy" and "lower alkoxy carbonyl".
The term "lower alkenyl" includes linear and branched alkenyl groups having one or more double bonds at an optional site(s) and having 2 to 10, preferably 2 to 8, more preferably 3 to 6 carbon atoms. Examples thereof include vinyl, propenyl, isopropenyl, butenyl, isobutenyl, prenyl, butadienyl, pentenyl, isopentenyl, pentadienyl, hexenyl, isohexenyl, hexadienyl, heptenyl, octenyl, nonenyl, decenyl and the like.
The above-described explanation about the "lower alkenyl" is equally applied to the lower alkenyl moiety in "aryl lower alkenyl", "lower alkenyloxy" and "aryl lower alkenyloxy".
The term "aryl" includes phenyl, naphthyl, anthryl and phenanthryl, and phenyl is especially preferred.
The above-described explanation about the "aryl" is equally applied to the aryl moiety in "aryl lower alkyl", "aryl lower alkylsulfonyl", "aryl lower alkoxy", "aryl lower alkenyl", "aryl lower alkenyloxy" and "arylsulfonyl".
The term "acyl" includes linear and branched chain aliphatic acyl groups having 1 to 10, preferably 1. to 6,

more preferably 1 to 4 carbon atoms, cyclic aliphatic acyl groupshaving 4 to 9, preferably 4 to 7 carbon atom sand aroyl. Examples thereof include formyl, acetyl, propionyl, butyryl, isobutyryl, valeryl, pivaloyl, hexanoyl, acryloyl, propioloyl, methacryloyl, crotonoyl, cyclopropylcarbonyl, cyclohexylcarbonyl, cyclooctylcarbonyl, benzoyl and the like.

The chain aliphatic acyl may be substituted with an aryl group(s), lower alkyl aryl group(s) and/or the like. The cyclic aliphatic acyl and aroyl may be substituted with a lower alkyl group(s).

The above-described explanation about the "acyl" is equally applied to the acyl moiety in "acyloxy".

The term "cycloalkyl" means a carbocyclic group having 3 to 8, preferably 3 to 6 carbon atoms. Examples thereof include cyclopropyl, cyclobutyl, cyclopentyl, cyclohexyl, cycloheptyl, cyclooctyl and the like.

The above-described explanation about the "cycloalkyl" is equally applied to the cycloalkyl moiety in "cycloalkyl lower alkyl".

The term "cycloalkenyl" includes those having one or more double bonds at an optional site(s) in the ring of the above-described cycloalkyl group. Examples thereof include cyclopropenyl, cyclobutenyl, cyclopentenyl, cyclohexenyl, cycloheptynyl, cyclooctynyl, cyclohexadienyl and the like.

The above-described explanation about the "cycloalkenyl" is equally applied to the cycloalkenyl moiety in "cycloalkenyl lower alkyl".

The phrase "$R^7$ and $R^8$ may optionally bind to adjacent carbon atoms in the ring and to form a ring together with the carbon atoms, which ring may optionally have a substituent(s)" means that

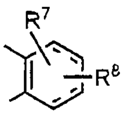

forms, for example,

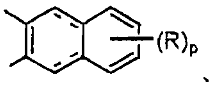 , 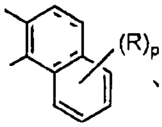 , 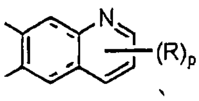 , 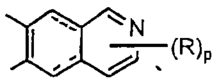 ,

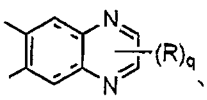 , 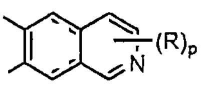 , 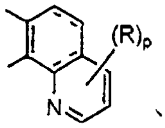 , 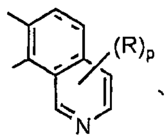 , 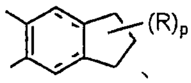 ,

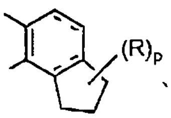 , 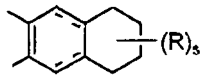 , 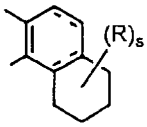 , 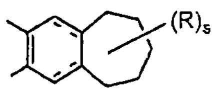 , 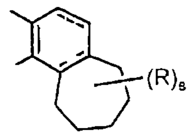

or the like

(wherein R represents lower alkyl, lower alkoxy, acyl, hydroxy lower alkyl, $SR^{11}$, $SOR^{11}$, $SO_2R^{11}$, $(CH_2)_rOR^{11}$, $(CH_2)_rCOOR^{11}$, $SO_2NR^{12}R^{13}$, $CONR^{12}R^{13}$, $(CH_2)_rNR^{12}R^{13}$ or $(CH_2)_rN(R^{12})COR^{13}$; p represents an integer of 0 to 3; q represents an integer of 0 to 2; s represents an integer of 0 to 4; and other symbols have the same meanings as described above. In cases where p, q and r are not less than 2, Rs may be the same or different).

The phrase "Z represents a crosslinkage composed of 2 to 5 atoms" means that Z is, for example, $-(CR^{9a}R^{9b})_2-$, $-(CR^{9a}R^{9b})_3-$, $-(CR^{9a}R^{9b})_4-$, $-(CR^{9a}R^{9b})_5-$, $-(CR^{9a}R^{9b})_2O-$, $-(CR^{9a}R^{9b})_2S-$, $-(CR^{9a}R^{9b})_2N(R^{10})-$, $-O(CR^{9a}R^{9b})_2-$, $-S(CR^{9a}R^{9b})_2-$, $-N(R^{10})(CR^{9a}R^{9b})_2-$ (wherein $R^{9a}$, $R^{9b}$ and $R^{10}$ have the same meanings as described above, and in cases where a plurality of $R^{9a}$s and $R^{9b}$s exist, $R^{9a}$s may be different, and $R^{9b}$s may be different) or the like.

In the present description, the term "solvate" includes, for example, solvates with organic solvents, hydrates and the like. In cases where a hydrate is formed, the compound may be coordinated with an optional number of water molecules.

Compound (I) includes pharmaceutically acceptable salts. Examples thereof include salts with an alkaline metal (such as lithium, sodium or potassium), an alkaline earth metal (such as magnesium, or carcium), an ammonium; an organic base, or an amino acid; and salts with an inorganic acid (such as hydrochloric acid, sulfuric acid, nitric acid, hydrobromic acid, phosphoric acid or hydroiodic acid) or organic acid (such as acetic acid, citric acid, lactic acid, tartaric acid, oxalic acid, malcic acid, fumaric acid, mandelic acid, glutaric acid, malic acid, benzoic acid, phthalic acid, benzenesulfonic acid,p-toluenesulfonic acid, methanesulfonic acid or ethanesulfonic acid). Salts with hydrochloric acid, phosphoric acid, tartaric acid, methanesulfonic acid or the like are especially preferred. These salts can be formed by an ordinary method.

Compound (I) is not restricted to a specific isomer, but includes all possible isomers and racemates.

Preferred examples of $R^1$ include hydrogen, lower alkyl, cycloalkyl lower alkyl, lower alkenyl, aryl lower alkyl, furyl lower alkyl and thienyl lower alkyl, and cyclopropylmethyl is especially preferred.

Preferred examples of $R^2$ include hydrogen, hydroxy, lower alkoxy, aryl lower alkoxy and acyloxy, and hydroxy is especially preferred.

Preferred examples of $R^3$ include hydrogen, hydroxy, lower alkoxy, aryl lower alkoxy and acyloxy, and hydroxy is especially preferred.

As for $R^4$ and $R^5$, those wherein $R^4$ is hydrogen, hydroxy, lower alkoxy or acyloxy, and $R^5$ is hydrogen, and those wherein $R^4$ and $R^5$ together form -O- or -S-are preferred.

Preferred examples of $R^6$ include hydrogen, lower alkyl, carboxy and lower alkoxycarbonyl, and hydrogen is especially Preferred.

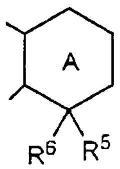 is preferably 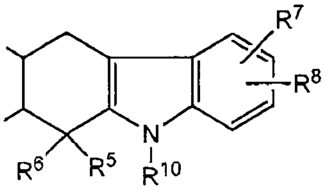 or 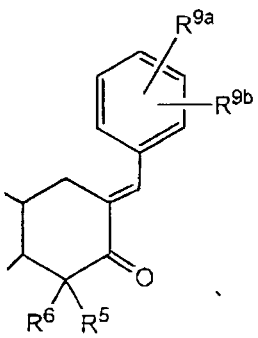 ,

especially wherein $R^7$and $R^8$ independently are hydrogen, halogen, nitro, $C_1$-$C_3$ alkyl, hydroxy, $C_1$-$C_3$ alkoxy, halogeno $C_1$-$C_3$ alkoxy, hydroxy $C_1$-$C_3$ alkyl, cyano, phenyl, isothiocyanato, $SR^{14}$, $SOR^{14}$, $SO_2R^{14}$ $(CH_2)_rOR^{14}$, $(CH_2)_rCOOR^{14}$, $SO_2NR^{15}R^{16}$, $CONR^{15}R^{16}$ or $(CH_2)rNR^{15}R^{16}$ (wherein $R^{14}$ is $C_1$-$C_3$ alkyl, $R^{15}$ and $R^{16}$ independently are hydrogen or $C_1$-$C_3$ alkyl, and r is an integer of 0 to 5), or $R^7$and $R^8$ together with the adjacent carbon atoms to which they are bound to form benzene ring, cyclopentane ring or cyclohexane ring; $R^{9a}$ and $R^{9b}$ independently arc hydrogen or $C_1$-$C_3$ alkyl; and $R^{10}$ is hydrogen or $C_1$-$C_3$ alkyl.

The compound (I) of the present invention can be produced by the methods such as those described in the above-described Patent Literatures 8, 9, 11 and 15, and in Non-patent Literature 2.

The "compound having an opioid δ receptor antagonist activity" may be any compound as long as it has a high affinity to δ receptor than to opioid receptors such as μ receptor and κ receptor (for example, the affinity to δ receptor is not less than 10 times, preferably not less than 20 times, more preferably not less than 30 times higher than the affinities to other opioid receptors), and has a δ receptor antagonist activity.

Examples thereof include 7-benzilidenenaltrexone (BNTX), [D-Ala$^2$, Leu$^5$, Cys$^6$] enkephalin (DALCE), naltriben, naltrindole 5'-isothiocyanate (5'-NTII), H-Tyr-Tic-Phe-Phc-OH(TIPP), naltrindole, N,N-diallyl-Tyr-Aib-Aib-Phc-Lcu-OH (ICl174,864), (N,N-bisallyl)-Tyr-Gly-Gly-ψ-($CH_2$S)-Phe-Leu-OH (IC1-154,129) and the like. Preferably, the compound having an opioid δ receptor antagonist activity is the above-described compound (I), a salt thereof, or a solvate of compound (I).

The term "constipation in which opioid μ receptor is involved" means the constipation induced by taking a compound having an opioid μ receptor agonist activity. Examples of the "compound having an opioid μ receptor agonist activity" include morphine, oxycodon, fentanyl, methadone, codeine, dihydrocodeine, hydromorphone, levorphanol, meperidine, propoxyphene, dextropropoxyphene and tramadol, as well as pharmaceutically acceptable salts thereof. The therapeutic and/or prophylactic agent according to the present invention is especially effective when the compound is morphine or oxycodon or a pharmaceutically acceptable salt thereof.

The compound of the present invention exhibits high amelioration effect against small intestine transit inhibition, the inhibitory action being induced by aμ receptor agonist administered to a patient suffering from a disease accompanying

pain (e.g., cancer pain (pain due to bone metastasis, compression of nerve, intracranial hypertension, infiltration into soft tissue, pain induced by constipation or twitching; pain of an internal organ, muscle or fascia; pain of lumbar or the vicinity of shoulder joint; chronic postsurgical pain), AIDS or the like), without substantially reducing the analgesic effect of the μ receptor agonist. Therefore, the compound of the present invention is useful as a therapeutic and/or propylactic agent against not only constipation, but also against irritable bowel syndrome or the like.
Especially, compound (I) has features such as high oral availability, low brain penetration, low toxicity and high stability in human plasma, and is very useful as a pharmaceutical.

The compound of the present invention may be administered before, after or simultaneously with the administration of the compound having the opioid μ receptor agonist activity.
The interval between the administration of the two drugs is not restricted. For example, in cases where the compound is administered after administration of the compound having the opioid μ receptor agonist activity, the compound of the present invention well functions if it is administered immediately after to about 3 days after, preferably immediately after to about 1 day after the administration of the compound having the opioid μ receptor agonist activity. In cases where the compound is administered before administration of the compound having the opioid μ receptor agonist activity, the compound of the present invention well functions if it is administered just before to about 1 day before, preferably just before to about 12 hours before the administration of the compound having the opioid μ receptor agonist activity.
When administering the compound of the present invention as a therapeutic or prophylactic agent for constipation, another therapeutic or prophylactic agent(s) for constipation may be used in combination. For example, a stimulant laxative(s) (such as sennoside and sodium picosulfate), and then an osmotic laxative(s) (lactulose) or a saline laxativc (s) (magnesium oxide) may be administered in combination.
When the compound of the present invention is administered to human as a therapeutic or prophylactic agent, the compound may be formulated in the form of powder, granules, tablets, capsules, balls, liquid or the like and may be administered orally, or the compound may be formulated in the form of injection solution, suppository, transdermal formulation, inhalant or the like and may be administered parenterally. The compound of the present invention may be formulated into a pharmaceutical preparation by mixing an effective amount of the compound of the present invention with a preferable additive(s) for pharmaceuticals such as vehicle(s), binder(s), wetting agent(s), disintegrator(s) and/or lubricant(s) according to its formulation.
The compound of the present invention may be in the form of a combination with the compound having the opioid μ receptor agonist activity and/or other therapeutic or prophylactic agent(s) for constipation, as well as various additives for pharmaceuticals as required.
Although the dose varies depending on the state of the disease, administration route and the age or body weight of the patient, the dose for an adult for oral administration is usually 1 μg to 10 g/day, preferably 0.1 to 2000 mg/day, and the dose for parenteral administration is usually 0.1 μg to 1 g/day, preferably 0.01 to 200 mg/day.

The present invention will now be described in more detail referring to examples and test examples. However, the present invention is not restricted thereto.

Examples

Test Example 1 Influence on Morphine-induced Inhibitory Action against Transport Competence of Gastrointestinal Tract

1) Test Compounds

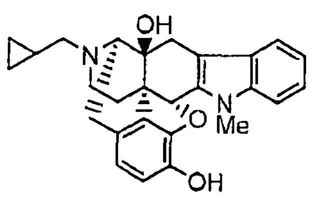

Compound 1

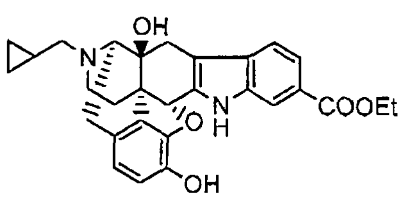

Compound 2

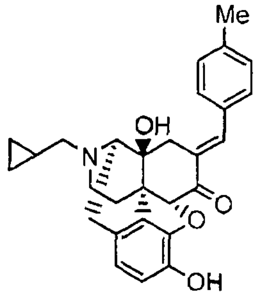

Compound 3

2) Preparation Method of Test Solutions

Test Solutions for Oral Administration:

After weighing each test compound, 0.5% methylcellulose (methylcellulose: Wako Pure Chemicals, water for injection: Otsuka Pharmaceutical Factory) was added, and the obtained mixture was well stirred to prepare solutions containing the test compound at final concentrations of 3 mg/mL, 1 mg/mL and 0.3 mg/mL, respectively.

Test Solutions for Subcutaneous Administration:

After weighing each test compound, 5% xylitol (Kylit injection (registered trademark), Otsuka Pharmaceutical Factory) was added and the mixture was well stirred to prepare solutions containing the test compound at a final concentration of 1 mg/mL. The solutions having concentrations of 0.3 mg/mL and 0.1 mg/mL, respectively, were prepared by diluting the solution having the concentration of 1 mg/mL.

3) Other Drugs

Morphine hydrochloride (Sankyo, hereinafter referred to as "morphine")

Preparation Method: After weighing, physiological saline was added and the mixture was well stirred to prepare a solution having a concentration of 0.3 mg/mL.

4) Animals

Crj:CD-1(ICR) mouse (SPF, male, 5-week old, body weight: 25.6-32.0 g (oral administration), 26.3-33.0 g (subcutaneous administration), Charles River Laboratories Japan)

5) Rearing Conditions

Temperature: 20-26°C

Humidity: 35-75%

Lighting Hours: 12 hours/day (7 to 19 o'clock)

Number of Ventilation: 15-25 times/hour

Feed: Solid Feed F-2 (Funabashi Farms), free feeding Drinking: tap water, free feeding

6) Test Groups

Oral Administration:

Control Group: 8 animals; Solvent-administered Group: 8 animals; Compound 1: 3, 10 and 30 mg/kg Groups: each 8 animals; Compound 2: 3, 10 and 30 mg/kg Groups: each 8 animals; Compound 3: 3, 10 and 30 mg/kg Groups: each 8 animals Subcutaneous Administration

Control Group: 8 animals; Solvent-administered Group: 8 animals; Compound 1: 3, 10 and 30 mg/kg Groups: each 8 animals; Compound 2: 3, 10 and 30 mg/kg Groups: each 8 animals; Compound 3: 3, 10 and 30 mg/kg Groups: each 8 animals

7) Administration

Administration volume: 10 mL/kg

Administration Method: Oral administration was carried out using a disposable syringe and an oral feeding tube. Subcutaneous administration was carried out using a disposable syringe and a disposable injection needle.

Administration Frequency: single

8) Test Procedure

Mice fasted overnight from the evening on one day before the test were used. Each test compound was administered, and 1 5 minutes later, morphine (3 mg/kg) was subcutaneously administered. 30 minutes after the administration of morphine, carbon powder (a suspension containing 5% of carbon powder suspended in 10% gum Arabic) was orally administered to each mouse in an amount of 0.1 mL, and the transfer rate (the distance which the carbon powder reached/the distance between the pylorus to cecum opening x 100) was measured at 30 minutes after the administration of carbon powder. To the solvent-administered group, a solvent (methylcellulose or xylitol) was administered in place of the test substance solution. To the control group, none of the test substance, morphine and the solvent was administered and the transfer rate of the carbon powder was measured.

9) Results

The results obtained by oral administration of the test substances are shown in Table 1 and in Fig. 1.

Table 1

| | Dose of Test Substance | Dose of Morphine | Transfer Rate of Carbon Powder (%) |
|---|---|---|---|
| Control Group | - | - | 57±1 |
| Solvent-administered Group (Solvent:0.5% methylcellulose) | - | 3 mg/kg(s.c.) | 25±2 |

(continued)

| | Dose of Test Substance | Dose of Morphine | Transfer Rate of Carbon Powder (%) |
|---|---|---|---|
| Compound 1-administered Group | 3 mg/kg | 3 mglkg(s.c.) | 38±2** |
| | 10 mg/kg | 3 mg/kg(s.c.) | 35±2** |
| | 30 mg/kg | 3 mg/kg(s.c.) | 40±2** |
| Compound 2-administered Group | 3 mg/kg | 3 mg/kg(s.c.) | 43±2** |
| | 10 mg/kg | 3 mg/kg(s.c.) | 38±2** |
| | 30 mg/kg | 3 mg/kg(s.c.) | 52±3** |
| **;<0.01 | | | |

The results obtained by subcutaneous administration of the test substances are shown in Table 2 and in Fig. 2.

Table 2

| | Dose of Test Substance | Dose of Morphine | Transfer Rate of Carbon Powder (%) |
|---|---|---|---|
| Control Group | - | - | 60±3 |
| Solvent-administered Group (Solvent:5% xylitol) | - | 3 g/kg(s.c.) | 27±1 |
| Compound 1-administered Group | 1 mg/kg | 3 mg/kg(s.c.) | 41±2** |
| | 3 mg/kg | 3 mg/kg(s.c.) | 43±3** |
| | 10 mg/kg | 3 mg/kg(s.c.) | 46±3** |
| Compound 2-administered Group | 1 mg/kg | 3 mg/kg(s.c.) | 35±3 |
| | 3 mg/kg | 3 mg/kg(s.c.) | 53±3** |
| | 10 mg/kg | 3 mg/kg(s.c.) | 55±2** |
| Compound 3-administered Group | 1 mg/kg | 3 mg/kg(s.c.) | 32±2 |
| | 3 mg/kg | 3 mg/kg(s.c.) | 43±1** |
| | 10 mg/kg | 3 mg/kg(s.c.) | 53±3** |
| **:<0.01 | | | |

As seen from the above-described results, compound (I) exhibited antagonistic action against the gastrointestinal transit inhibition, which was induced by administration of morphine.

Example 1 Synthesis of 17-cyclopropylmethyl-6,7-didehydro-4,5α-cpoxy-3,14β-dihydroxy-6'-carboxy-6,7-2',3'-indolomorphinan (4)

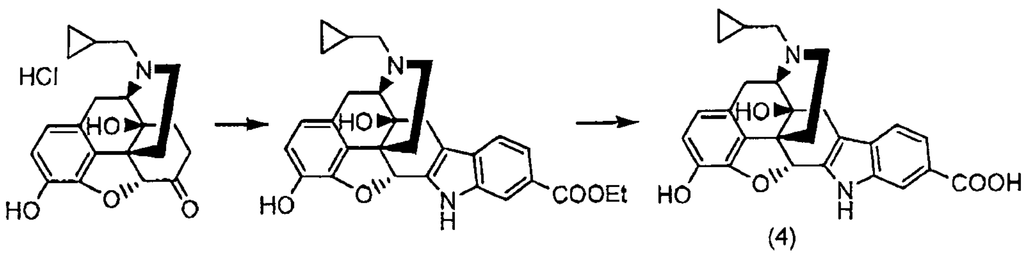

(4)

(First Step) 17-cyclopropylmethyl-6,7-didehydro-4,5α-epoxy-3,14β-dihydroxy-6'-ethoxycarbonyl-6,7-2',3'-indolomorphinan

Known naltrexone hydrochloric acid salt (500 mg, 1.32 mmol) and o-laydrazinobenzoic acid (221 mg, 1.46 mmol) were suspended in 3 ml of ethanol, and the suspension was heated at 50°C with stirring. To the mixture, methanesulfonic acid (0.86 mL, 13.2 mmol) solution in 2 mL of ethanol was slowly added dropwise for 10 minutes. After completion of the dropwise addition, the mixture was stirred for 2 hours under reflux. After allowing the mixture to cool to room temperature, saturated aqueous sodium hydrogen carbonate solution and ethyl acetate were added to the reaction solution. The organic layer was separated and washed with water and then with saturated brine. After drying the organic layer over sodium sulfate, the solvent was evaporated. The residue was purified by silica gel column chromatography (chloroform:methanol = 99:1) to obtain 379 mg (59%) of the captioned compound as pale yellow solid.
NMR(300MHz, $CDCl_3$)
δ 0.14-0.18 (m, 2H), 0.55-0.59 (m, 2H), 0.89 (m, 1H), 1.41 (t, 3H, J = 6.9 Hz),1.75 (d, 1H, J = 11.4 Hz), 2.20-2.91 (m, 8H), 3.10 (d, 1H, J = 18.6 Hz), 3.38 (d, 1H, J = 6.3 Hz), 4.38 (q, 2H, J = 6.9 Hz), 5.5 (br s, 1H), 5.69 (s, 1H), 6.46 (d, 1H, J = 8.1 Hz), 6.55 (d, 1H, J = 8.1Hz), 7.34 (d, J = 8.4 Hz), 7.67 (d, J = 8.4 Hz), 7.92 (s, 1H), 8.36 (s, 1H).

(Second Step) 17-cyclopropylmethyl-6,7-didehydro-4,5α,-epoxy-3,14β-dihydroxy-6'-carboxy-6,7-2',3'-indolomorphinan

To a solution of the compound (654 mg, 1.20 mmol) obtained in Step 1 in methanol (2.4 mL), 2 mol/L aqueous sodium hydroxide solution (2.4 mL) was added, and the mixture was stirred for 1 hour under reflux. After allowing the reaction solution to cool to room temperature, the reaction solution was diluted with methanol, and its pH was adjusted to 6.0 with dilute hydrochloric acid. The precipitated crystals were collected by filtration, washed with water and dried to obtain 534 mg (97%) of the captioned compound as colorless crystals.
NMR (300MHz, d6-DMSO)
δ 0.14-0.18 (m, 2H), 0.48-0.54 (m, 2H), 0.90 (m, 1H), 1.59 (d, 1H, J = 11.7 Hz), 2.09-2.82 (m, 8H), 3.07 (d, 1H, J = 18.6 Hz), 5.55 (s, 1H), 6.49 (d, 1H, J = 7.8 Hz), 6.52 (d, 1H, J = 7.8Hz), 7.42 (d, J = 8.4 Hz), 7.55 (dd, J = 1.5, 8.4 Hz), 7.97 (d, J = 1.5 Hz, 1H), 8.98 (br s, 1H), 11.54 (s, 1H).

Test Example 2 Influence on Morphine-induced Small Intestine Transit Inhibition

1) Preparation of Test Meal (Pigment)
Using aqueous 0.5 w/v% Evans blue solution, 2.5 w/v% carboxymethylcellulose salt solution was prepared and used as the test meal.
2) Animals Used
Male Wistar rats (Crj. Wistar, Charles River Laboratories Japan, 6 to 7-week old) were used. The rats were fasted from not less than 20 hours before the beginning of the test, and water was given *ad libitum.*
3) Test substance and Medium
The compound (I-1) synthesized in Example 1 was dissolved in a solvent (DMAA/Solutol/5% meglumine = 15/15/70)
DMAA: N,N-dimethylacetamide (Kanto Chemical)
Solutol: Solutol (registered trademark) HS15 (BASF)
Meglumine: D(-)-N-methylglucamin (Merck)
Morphine hydrochloric acid salt (Dainippon Pharma) was dissolved in normal saline.
All of the test substance, solvent and morphine were administered in avolume of 2 mL/kg.
4) Test Method
Each test substance in an amount of 0.03, 0.1, 0.3, 1 or 3 mg/kg (Test Substance-administered Groups) or the above-described solvent (Solvent-administered Group) was subcutaneously administered, and 75 minutes later, 3 mg/kg of morphine was subcutaneously administered to all groups. To the control group, the above-described solvent was subcutaneously administered, and 75 minutes later, normal saline was administered.
The test meal in an amount of 2 mL/rat was orally administered at 30 minutes after the administration of morphine. 15 minutes after the administration of the test meal (at 120 minutes after the administration of the test substance), the portion from the vicinity of cardia in the esophagus to the ileocecum was extirpated. The distance between the cardia and the ileocecum (full length of small intestine), and the distance up to the tip of the pigment (moving distance of the pigment) were measured.
5) Data Processing

$$\text{Transport Rate (\%)} = (\text{Moving Distance of Pigment (cm)}/\text{Full Length of Small Intestine (cm)}) \times 100$$

$$\text{M.P.E.(\%)} = \{(A(\%) - B(\%)\}/(C(\%) - B(\%))\} \times 100$$

(A: Transport Rate (%) in Small Intestine of Each Individual in Test Substance-administered Group;
B: Mean Transport Rate (%) in Small Intestine of Solvent-administered Group
C: Mean Transport Rate (%) in Small Intestine of Control Group

$ED_{50}$ was calculated using %MPE, by the inverse estimation of the regression of SAS program taking the value of the control group as 100%. As the significant test, Dunnett's test was used.
6) Results
The compound of the present invention exhibited antagonistic action against the morphine-induced small intestine transit inhibition, and the $ED_{50}$ value was 0.29 mg/kg.

Formulation Example 1

Granules comprising the following components are prepared:

| Components | |
|---|---|
| Compound of Formula (I) | 10 mg |
| Lactose | 700 mg |
| Corn Starch | 274 mg |
| HPC-L | 16 mg |
| | 1000 mg |

The compound of Formula (I) and lactose are made to pass through a 60-mesh sieve. Corn starch is made to pass through a 120-mesh sieve. These are mixed with a V-shaped mixer, An aqueous HPC-L (low viscosity hydroxypropylcellulose) solution is added to the mixed powder, and the resulting mixture is subjected to kneading, granulation (extrusion granulation, pore size 0.5 to 1 mm) and drying. The obtained dried granules arc made to pass through a vibrating sieve (12/60-mesh) to obtain granules.

Formulation Example 2

Granules for capsulation containing the following components are prepared:

| Components | |
|---|---|
| Compound of Formula (I) | 15 mg |
| Lactose | 90 mg |
| Corn Starch | 42 mg |
| HPC-L | 3 mg |
| | 150 mg |

The compound of Formula (I) and lactose are made to pass through a 60-mesh sieve. Corn starch is made to pass through a 120-mesh sieve. These are mixed and an aqueous HPC-L solution is added to the mixed powder, and the resulting mixture is subjected to kneading, granulation and drying. After regulating the particle size of the dried granules, 150 mg thereof is filled in a hardness 4 gelatin capsule.

Formulation Example 3

Tables containing the following components are prepared:

| Components | |
|---|---|
| Compound of Formula (1) | 10 mg |
| Lactose | 90 mg |
| Microcrystalline Cellulose | 30 mg |
| CMC-Na | 15mg |
| Magnesium Stearate | 5 mg |
| | 150 mg |

The compound of Formula (1), lactose, microcrystalline cellulose and CMC-Na (carboxymethylcellulose sodium salt) were made to pass through a 60-mesh sieve and mixed. The mixed powder is mixed with magnesium stearate to obtain mixed powder for tableting. The mixture is directly subjected to tablet making to obtain 150 mg of tablets.

Formulation Example 4

The following components were mixed under heat, and the mixture was sterilized to obtain an injection solution.

| | |
|---|---|
| Compound of the Present Invention | 3 mg |
| Nonioinic Surfactant | 15 mg |
| Purified Water for Injection | 1 ml |

Industrial Availability

The compound of the present invention can be a pharmaceutical useful for the therapy or prophylaxis of constipation.

Brief Description of the Drawings

Fig. 1 is a graph showing the influence on the gastrointestinal transit when the compound of the present invention was orally administered.
Fig. 2 is a graph showing the influence on the gastrointestinal transit when the compound of the present invention was subcutaneously administered.

## Claims

1. A therapeutic and/or prophylactic agent for constipation in which opioid μ receptor is involved, said agent comprising a compound having an opioid δ receptor antagonist activity.

2. A therapeutic and/or prophylactic agent for constipation induced by a compound having an opioid μ receptor agonist activity, said agent comprising a compound having an opioid δ receptor antagonist activity.

3. The therapeutic and/or prophylactic agent for constipation according to claim 1 or 2, wherein said compound having the opioid δ receptor antagonist activity has a higher affinity to opioid δ receptor than to opioid μ receptor.

4. The therapeutic and/or prophylactic agent for constipation according to claim 1 or 2, wherein said compound having the opioid δ receptor antagonist activity is represented by Formula (I):

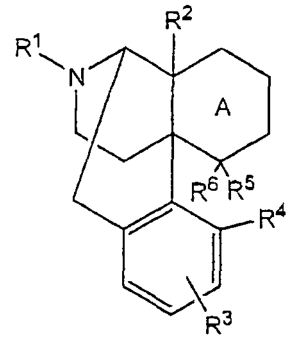

(I)

(wherein $R^1$ represents hydrogen, lower alkyl, cycloalkyl lower alkyl, cycloalkenyl lower alkyl, lower alkenyl, aryl, aryl lower alkyl, furyl lower alkyl or thienyl lower alkyl;
$R^2$ and $R^3$ independently represent hydrogen, hydroxy, lower alkoxy, lower alkenyloxy, aryl lower alkoxy, aryl lower alkenyloxy, acyloxy or lower alkoxy lower alkoxy;
$R^4$ represents hydrogen, hydroxy, lower alkoxy or acyloxy;
$R^5$ represents hydrogen;
$R^4$ and $R^5$ may optionally together form -O-, -S- or $-CH_2-$;
$R^6$ represents hydrogen, lower alkyl, lower alkenyl, hydroxy lower alkyl, lower alkoxy lower alkyl, lower alkoxycarbonyl lower alkyl, aryl lower alkyl, aryl lower alkenyl, carboxy or lower alkoxycarbonyl;

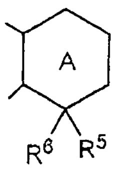

is

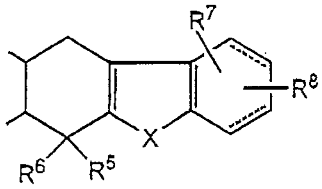

,

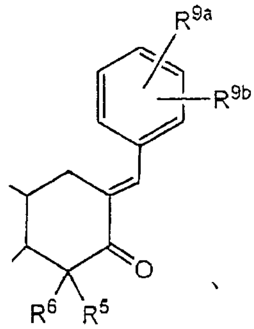

,

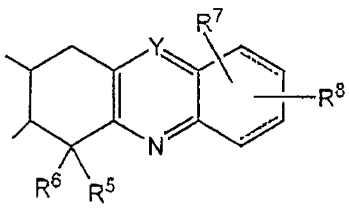

or

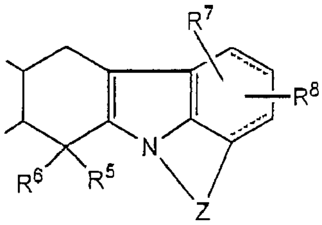

(wherein X represents -O-. -S-, -CH=CH- or $-N(R^{10})-$;
$R^7$, $R^8$, $R^{9a}$ and $R^{9b}$ independently represent hydrogen, halogen, nitro, lower alkyl, hydroxy, lower alkoxy, halogeno lower alkyl, hydroxy lower alkyl, halogeno lower alkoxy, hydroxy lower alkoxy, cyano, phenyl, isothiocyanato, $SR^{11}$, $SOR^{11}$, $SO_2R^{11}$, $(CH_2)_rOR^{11}$, $(CH_2)_rCOOR^{11}$, $SO_2NR^{12}R^{13}$, $CONR^{12}R^{13}$, $(CH_2)_rNR^{12}R^{13}$ or $(CH_2)_rN(R^{12})COR^{13}$;
$R^7$ and $R^8$ may optionally bind to adjacent carbon atoms in the ring to form a ring together with the carbon atoms, which ring may have a substituent(s);
broken lines represent presence or absence of a bond, in cases where the broken lines represent absence of the bond and, $R^7$ and $R^8$ may optionally together form =O;
r represents an integer of 0 to 5;
$R^{10}$ represents hydrogen, lower alkyl, lower alkenyl, aryl lower alkyl, aryl lower alkenyl, acyl, lower alkylsulfonyl, arylsulfonyl, aryl lower alkylsulfonyl or acyl;
Y represents -N- or -CH-;
Z represents a crosslinkage composed of 2 to 5 atoms;

$R^{11}$ represents hydrogen or lower alkyl;
$R^{12}$ and $R^{13}$ independently represent hydrogen, lower alkyl or cycloalkyl lower alkyl) or a pharmaceutically acceptable salt thereof or a solvate of either.

**5.** The therapeutic and/or prophylactic agent according to claim 4, wherein $R^1$ is cycloalkyl lower alkyl;
$R^2$ and $R^3$ are hydroxy;

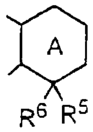

is

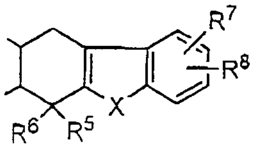

or

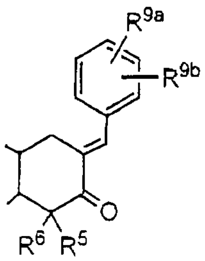

$R^4$ and $R^5$ together form -0-;
$R^6$ is hydrogen;
$R^7$, $R^8$, $R^{9a}$ and $R^{9b}$ independently are hydrogen, lower alkyl, carboxy or lower alkoxycarbonyl;
$R^{10}$ is hydrogen or lower alkyl.

**6.** The therapeutic and/or prophylactic agent according to any one of claims 2 to 5, wherein said compound having the opioid μ receptor agonist activity is morphine, oxycodone or a pharmaceutically acceptable salt thereof.

**7.** Use of a compound having an opioid δ receptor antagonist activity for the therapy and/or prophylaxis of constipation in which opioid μ receptor is involved.

**8.** Use of a compound having an opioid δ receptor antagonist activity for the therapy and/or prophylaxis of constipation induced by a compound having an opioid μ receptor agonist activity.

**9.** Use of the compound represented by Formula (I) recited in claim 4 or a pharmaceutically acceptable salt thereof or a solvate of either for the therapy and/or prophylaxis of constipation in which opioid μ receptor is involved.

**10.** Use of a compound represented by Formula (1) recited in claim 4 or a pharmaceutically acceptable salt thereof or a solvate of either for the therapy and/or prophylaxis of constipation induced by a compound having an opioid μ receptor agonist activity.

**11.** A therapeutic and/or prophylactic method for constipation in which opioid μ receptor is involved, said method comprising administering a compound having an opioid δ receptor antagonist activity.

**12.** A therapeutic and/or prophylactic method for constipation induced by a compound having an opioid μ receptor agonist activity, said method comprising administering a compound having an opioid δ receptor antagonist activity.

**13.** A therapeutic and/or prophylactic method for constipation in which opioid μ receptor is involved, said method comprising administering the compound represented by Formula (I) recited in claim 4 or a pharmaceutically acceptable salt thereof or a solvate of said compound or the pharmaceutically acceptable salt thereof.

**14.** A therapeutic and/or prophylactic method for constipation induced by a compound having an opioid μ receptor agonist activity, said method comprising administering the compound represented by Formula (I) recited in claim 4 or a pharmaceutically acceptable salt thereof or a solvate of either.

**15.** An analgesic comprising a compound having an opioid μ receptor agonist activity in combination with the compound represented by Formula (I) recited in claim 4 or a pharmaceutically acceptable salt thereof or a solvate of either in an amount effective for the therapy and/or prophylaxis of constipation induced by said compound having the opioid μ receptor agonist activity.

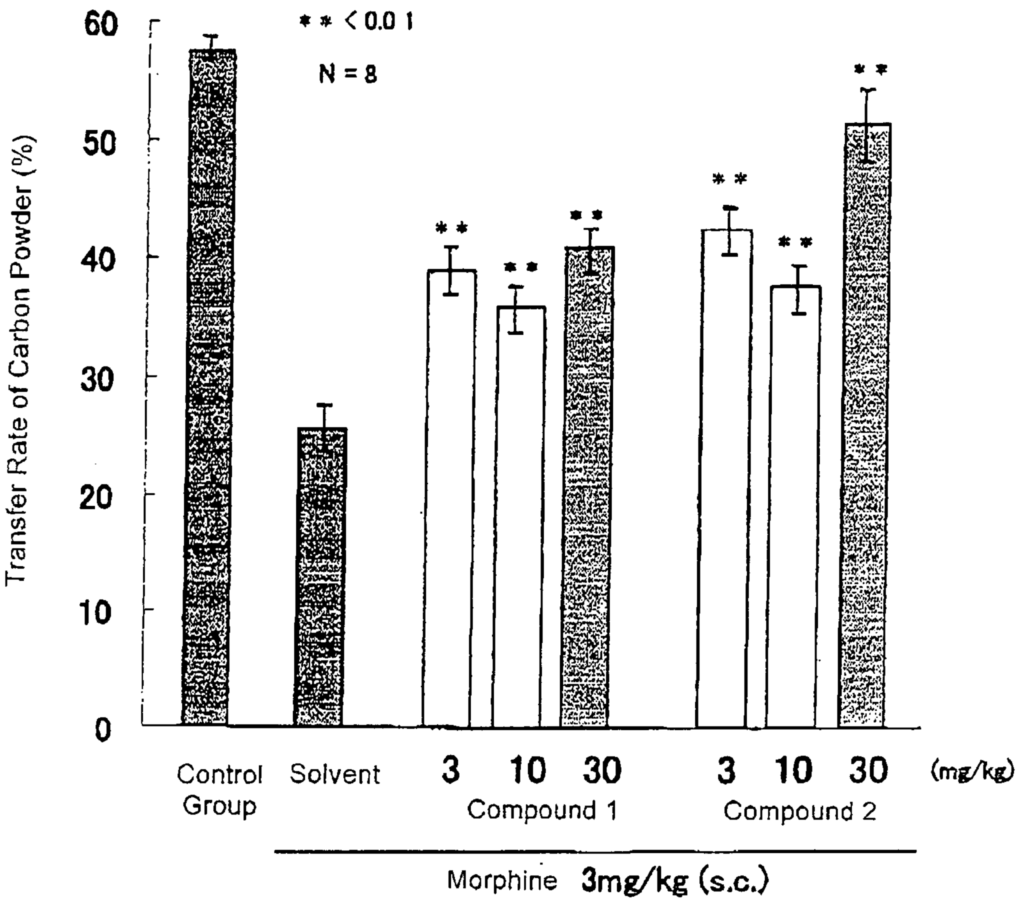
Transfer Rate of Carbon Powder (%)
60
50
40
30
20
10
0
** < 0.01
N = 8
**
**
**
**
**
**
Control Group
Solvent
3 10 30
Compound 1
3 10 30
Compound 2
(mg/kg)
Morphine 3mg/kg (s.c.)

Fig.1

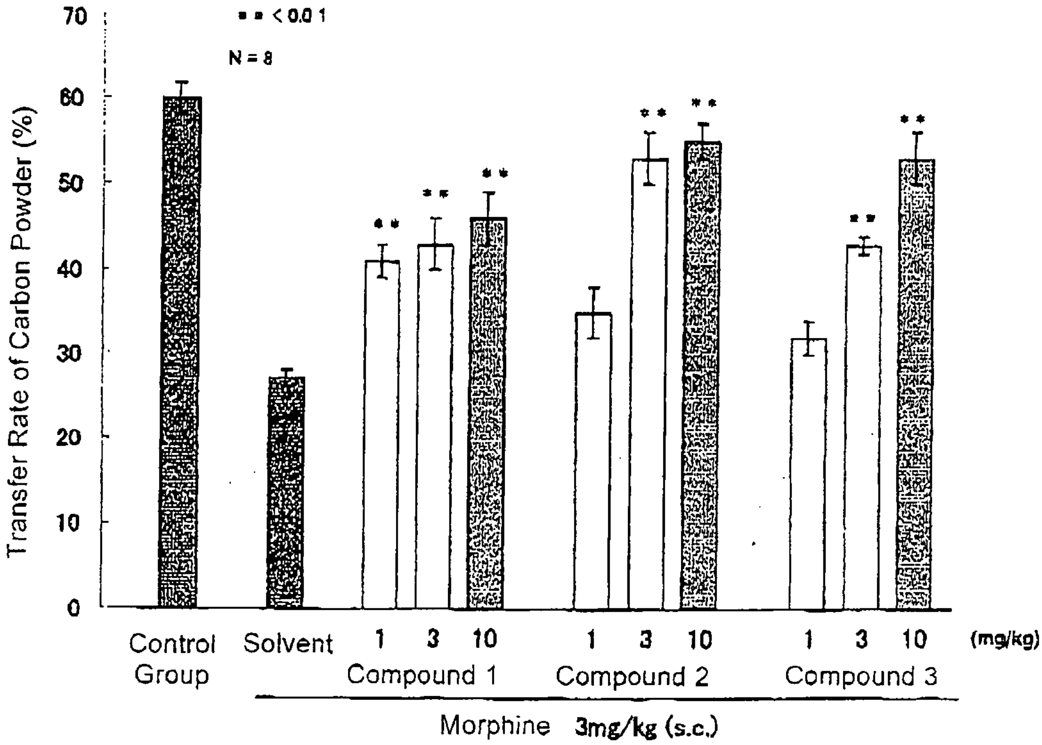

70
60
50
40
30
20
10
0
** < 0.0 1
N = 8
Transfer Rate of Carbon Powder (%)
**
Control Group
Solvent
1 3 10
Compound 1
1 3 10
Compound 2
1 3 10
Compound 3
(mg/kg)
Morphine 3mg/kg (s.c.)


**Fig.2**

**INTERNATIONAL SEARCH REPORT**

International application No.
PCT/JP2005/022822

A. CLASSIFICATION OF SUBJECT MATTER
***A61K45/00***(2006.01), ***A61K31/485***(2006.01), ***A61P1/10***(2006.01)

According to International Patent Classification (IPC) or to both national classification and IPC

B. FIELDS SEARCHED

Minimum documentation searched (classification system followed by classification symbols)
***A61K45/00***(2006.01), ***A61K31/485***(2006.01), ***A61P1/10***(2006.01)

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2006 |
| Kokai Jitsuyo Shinan Koho | 1971-2006 | Toroku Jitsuyo Shinan Koho | 1994-2006 |

Electronic data base consulted during the international search (name of data base and, where practicable, search terms used)
BIOSIS(STN), CAplus(STN), EMBASE(STN), MEDLINE(STN), REGISTRY(STN), PubMed, Science Direct, JMEDPlus(JOIS), JST7580(JOIS), JSTPlus(JOIS)

C. DOCUMENTS CONSIDERED TO BE RELEVANT

| Category* | Citation of document, with indication, where appropriate, of the relevant passages | Relevant to claim No. |
|---|---|---|
| X | Youn Seon Choi, Opioid Antagonists: A Review of Their Role in Palliative Care, Focusing on Use in Opioid-Related Constipation, Journal of Pain and Symptom Management, 2002, Vol.24, No.1, pages 71-90, full text | 1,2,6 |
| X<br>Y | WO 2004/007503 A1 (TORAY IND. INC.), 22 January, 2004 (22.01.04), Full text & EP 1522542 A1 & JP 2004-521168 A | 15<br>3-5 |
| Y | JP 2003-528819 A (ADOLOR CORP.), 30 September, 2003 (30.09.03), Full text & WO 2001/37785 A2 & EP 1244447 A2 | 3-5 |

☐ Further documents are listed in the continuation of Box C.  ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 02 March, 2006 (02.03.06) | 14 March, 2006 (14.03.06) |

| Name and mailing address of the ISA/ | Authorized officer |
|---|---|
| Japanese Patent Office | |
| Facsimile No. | Telephone No. |

Form PCT/ISA/210 (second sheet) (April 2005)

**INTERNATIONAL SEARCH REPORT**

International application No. PCT/JP2005/022822

**Box No. II Observations where certain claims were found unsearchable (Continuation of item 2 of first sheet)**

This international search report has not been established in respect of certain claims under Article 17(2)(a) for the following reasons:

1. [X] Claims Nos.: 7-14
because they relate to subject matter not required to be searched by this Authority, namely:
Claims 7 to 14 pertain to methods for treatment of the human body by therapy and thus relate to a subject matter which this International Searching Authority is not required, under the provisions of Article 17(2)(a)(i) of the PCT and Rule 39.1(iv) of the Regulations under the PCT, to search.

2. [ ] Claims Nos.:
because they relate to parts of the international application that do not comply with the prescribed requirements to such an extent that no meaningful international search can be carried out, specifically:

3. [ ] Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:
Claims 1-6 pertain to a subject matter in which a compound having opioid δ receptor antagonistic activity, such as, e.g., a compound represented by the formula (I), is used as a therapeutic and/or preventive agent for constipation in which an opioid μ receptor participates. Claim 15 pertains to an analgesic comprising a combination of a compound having opioid μ receptor agonistic activity and a compound represented by the formula (I).

A medicine containing a compound represented by the formula (I), which is a matter common between claims 1-6 and claim 15, is known as apparent from the fact that it is described in, e.g., the document (International Publication

(continued to extra sheet)

1. [ ] As all required additional search fees were timely paid by the applicant, this international search report covers all searchable claims.
2. [X] As all searchable claims could be searched without effort justifying an additional fee, this Authority did not invite payment of any additional fee.
3. [ ] As only some of the required additional search fees were timely paid by the applicant, this international search report covers only those claims for which fees were paid, specifically claims Nos.:

4. [ ] No required additional search fees were timely paid by the applicant. Consequently, this international search report is restricted to the invention first mentioned in the claims; it is covered by claims Nos.:

**Remark on Protest** the

[ ] The additional search fees were accompanied by the applicant's protest and, where applicable, payment of a protest fee..

[ ] The additional search fees were accompanied by the applicant's protest but the applicable protest fee was not paid within the time limit specified in the invitation.

Form PCT/ISA/210 (continuation of first sheet (2)) (April 2005)

INTERNATIONAL SEARCH REPORT

International application No.
PCT/JP2005/022822

Continuation of Box No.III of continuation of first sheet(2)

No. 2004/7503, pamphlet).

Consequently, that common matter is within the scope of the prior art and cannot be regarded as a special technical feature. Any other matter common among all the claims is not considered to be as a special technical feature. Therefore, the number of inventions included in this application is 2.

<With respect to subject matter for search>

Claims 1-3 pertain to a therapeutic and/or preventive agent for "constipation in which an opioid μ receptor participates," such as that "induced by a compound having opioid μ receptor antagonistic activity," which contains as an active ingredient a compound defined by the desirable property, i.e., "opioid δ receptor antagonistic activity."

Claims 1-3 involve all compounds having such property. However, it is considered that the compounds supported by the description in the meaning of Article 6 of the PCT and disclosed in the meaning of Article 5 of the PCT are limited to an extremely small part of the compounds claimed.

The term "opioid δ receptor antagonist" cannot be used to specify the scope of compounds having such property, even when technical common sense at the time of the filing of this application is taken into account. Consequently, claims 1-3 do not comply with the requirement of clearness as provided for in Article 6 of the PCT.

Therefore, a search was made for the relationship between an opioid δ receptor antagonist and constipation and for the constipation remedy containing as an active ingredient any of the compounds specified in the description and the compounds shown in claims 4 and 5.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 8303197 A
- WO 9922737 A
- WO 01032180 A
- WO 9825613 A
- JP H597806 A
- WO 01037785 A
- WO 0142207 A
- WO 8900995 A
- WO 9531463 A
- WO 9407896 A
- WO 9711948 A
- WO 0242309 A
- WO 2004007503 A
- WO 9831684 A
- WO 9414445 A
- WO 9107966 A
- US 6271239 B
- WO 9513071 A
- WO 9321188 A
- US 6476044 B


### Non-patent literature cited in the description


- *Journal of Pharmacology and Experimental Therapeutics,* 2002, vol. 300 (1), 118-123
- *Heterocycles,* 1997, vol. 45, 2109-2112
- *Journal of Medicinal Chemistry,* 1998, vol. 41, 4177-4180